# EUROPEAN PATENT APPLICATION

(11) **EP 4 230 736 A1**
(43) Date of publication of application: **23.08.2023**
(21) Application number: 21879947.6
(22) Date of filing: 06.10.2021
(51) Int. Cl.: C12N 15/09, C12Q 1/6827, C12Q 1/686, C12Q 1/6876

(54) **METHOD FOR DETECTING GENETIC POLYMORPHISM**

(30) Priority: 14.10.2020 JP 2020172944
(71) Applicant: Shimadzu Corporation, Kyoto-shi Kyoto 604-8511 (JP); National Cerebral and Cardiovascular Center, Suita-shi, Osaka 564-8565 (JP)
(72) Inventor: KAWAKAMI Daisuke, Kyoto-shi, Kyoto 604-8511 (JP); IHARA Masafumi, Kishibe-Shimmachi, Suita-shi, Osaka 564-8565 (JP)
(74) Representative: Müller-Boré & Partner Patentanwälte PartG mbB
(86) International application number: PCT/JP2021/036962
(87) International publication number: WO 2022/080207

(57) **Abstract**

The present invention chiefly aims to provide a new method of detecting or determining ApoE genetic polymorphism, that is rapid and less invasive to the subject.

The present invention includes, for example, a method for detecting a genetic polymorphism of apolipoprotein E present in genomic DNA collected from a subject, comprising the following steps 1 to 3:
1. a step of preparing a specimen with DNA released from saliva;
2. a step of adding the followings to the specimen containing the DNA and then mixing:
(1) a PCR enzyme, (2) a PCR primer pair for amplifying a nucleic acid fragment of the apolipoprotein E gene, and
(3) a fluorescent labeled probe set which has oligonucleotides for detecting the genetic polymorphism of apolipoprotein E; and

3. a step of performing PCR, and measuring the fluorescence intensities according to the genetic polymorphism of apolipoprotein E from the PCR product.

## Description

### Technical Field

The present invention belongs to the technical field of genetic polymorphism detection methods. The present invention relates to a method for detecting genetic polymorphism in apolipoprotein E (hereinafter also referred to as "ApoE"). In detail, the present invention relates to a method for detecting ApoE genetic polymorphism from saliva and other exocrine fluids.

### Background of the Invention

ApoE is a protein with a molecular weight of 34,200 Da, consisting of 299 amino acids. The ApoE gene has ε2, ε3, and ε4 alleles, and there are three major isoforms ApoE2, ApoE3, and ApoE4 encoded by the respective alleles. These differ in two amino acid residues at positions 112 and 158: Cys/Cys for ApoE2, Cys/Arg for ApoE3, and Arg/Arg for ApoE4. Of these, ApoE3, in which the 112th amino acid residue is Cys and the 158th amino acid residue is Arg, is the wild type and most frequent; ApoE2 and ApoE4 are mutant forms.

ApoE is a molecule involved in lipid metabolism, including cholesterol transport and lipoprotein metabolism, and is involved in the activation of lipid metabolism enzymes such as liver lipase, lipoprotein lipase, and lecithin-cholesterol acyltransferase.

Many risk genes have been reported to be involved in the development of Alzheimer's disease (AD) and cognitive dysfunction (e.g., MCI), of which ApoE is the most potent risk gene. Additionally, it has been reported that the frequency of ApoE4 is high and that of ApoE2 is low in older-onset AD. Furthermore, it is also known, for example, that the frequency of ApoE4 is almost the same in younger-onset and older-onset AD in women, while it is higher in older-onset AD than in younger-onset AD in men; that ApoE4/4 accelerates the age of onset of AD by about 5 to 10 years; that AD patients having ApoE4/4 are less responsive to AD drugs and are lower in rate of glucose metabolism; and that brain atrophy is more likely to progress in the order of ApoE4/4, ApoE3/4, and ApoE3/3.

It is also known that ApoE4 is more frequent in other dementias, such as vascular dementia (VD) and Lewy body disease, and that ApoE2 is more frequent in patients with cerebral hemorrhage due to cerebral amyloid angiopathy.

In addition, high frequencies of ApoE4 have been reported in neuropsychiatric disorders such as Parkinson's disease, schizophrenia, depression, anxiety disorders, and epilepsy.

As mentioned above, ApoE genetic polymorphism is considered an important factor in knowing the risk of developing AD and other diseases.

Conventionally, when diagnosing or predicting the onset of AD, for example, it is common to make a judgment based on MRI (Magnetic Resonance Imaging: MRI) or CT (Computed Tomography) images. However, taking MRI images requires a certain amount of time and places a heavy burden on the patient. Therefore, research is being conducted on methods to diagnose and predict the onset of AD using information on ApoE genetic polymorphism, which can be determined more quickly.

To date, measurement of ApoE genetic polymorphism has usually been performed by collecting blood samples from subjects. For example, Patent Document 1 discloses a method for detecting ApoE4 or its fragments in blood samples. In non-patent document 1, ApoE genetic polymorphism is detected using real-time PCR. Such methods for detecting ApoE genetic polymorphism in blood samples are highly invasive and still impose a high burden on the subject.

### Prior Art

### Patent Document

Patent Document 1: JP, 2019-536007, A

### Non-patent document

Non-Patent Document 1: Clinical Chemistry 45, No. 7, 1999, pp. 1094-1097

### Summary of the Invention

### Problem to be Solved by the Invention

The measurement of ApoE genetic polymorphism to date has usually been performed by taking blood samples from the subject, which is highly invasive and, like diagnostic imaging, still places a high burden on the subject.

The present invention chiefly aims to provide a new method of detecting or determining ApoE genetic polymorphism, that is rapid and less invasive to the subject, and can solve the above problems. It also provides a kit and a system therefor.

### Means for Solving the Problem

The present inventors, after earnest studies, found that ApoE genetic polymorphism can be easily and rapidly detected from exocrine fluids such as saliva, thereby solving the above problem, and they have completed the present invention.

The present invention, for example, can be described as follows.
[1] A method for detecting a genetic polymorphism of apolipoprotein E present in genomic DNA collected from a subject, comprising the following steps 1 to 3:
1. a step of releasing a DNA from exocrine fluid or mucus containing epithelial cells collected from a subject and preparing a specimen containing the DNA;
2. a step of adding the followings to the specimen containing the DNA and then mixing:
   (1) a PCR enzyme,
   (2) a PCR primer pair for amplifying a nucleic acid fragment of the apolipoprotein E gene containing a codon encoding the 112th or 158th amino acid residue (Cys or Arg) of apolipoprotein E, and
   (3) a set consisting of a fluorescent labeled probe which has an oligonucleotide that binds to a nucleic acid fragment of the apolipoprotein E gene, containing a codon encoding wild-type Cys which is the 112th amino acid residue of apolipoprotein E, and a fluorescent labeled probe which has an oligonucleotide that binds to a nucleic acid fragment of the apolipoprotein E gene, containing a codon encoding mutant Arg which is the 112th amino acid residue of apolipoprotein E, wherein the fluorescent dyes used for labeling are different from each other,
      or a set consisting of a fluorescent labeled probe which has an oligonucleotide that binds to a nucleic acid fragment of the apolipoprotein E gene, containing a codon encoding wild-type Arg which is the 158th amino acid residue of apolipoprotein E, and a fluorescent labeled probe which has an oligonucleotide that binds to a nucleic acid fragment of the apolipoprotein E gene, containing a codon encoding mutant Cys which is the 158th amino acid residue of apolipoprotein E, wherein the fluorescent dyes used for labeling are different from each other; and
3. a step of performing PCR on the said mixture and measuring the fluorescence intensity from the PCR product corresponding to the 112th or 158th amino acid residue of apolipoprotein E of the said subject.

[2] The method for detecting a genetic polymorphism according to the [1] above, wherein DNA is released by using a surfactant and protease K in the step 1 mentioned above.
[3] The method for detecting a genetic polymorphism according to the [2] above, wherein the surfactant is sodium dodecyl sulfate.
[4] The method for detecting a genetic polymorphism according to any one of the [1] to [3] above, wherein Tris hydrochloric acid buffer solution containing potassium chloride, magnesium chloride, and dNTP mix is further added and mixed with the above specimen.
[5] The method for detecting a genetic polymorphism according to any one of the [1] to [4] above, further comprising a step of adding and mixing with the above specimen a substance which binds to substances that inhibit PCR, which are a biologically derived negatively charged substance that adsorbs to PCR enzymes and a biologically derived positively charged substance that adsorbs to DNA, thereby neutralizing the PCR inhibitory action of the negatively charged substance and the positively charged substance.
[6] The method for detecting a genetic polymorphism according to any one of the [1] to [5] above, wherein the exocrine fluid or mucus is saliva.
[7] The method for detecting a genetic polymorphism according to the [6] above, wherein the saliva is collected by a cotton swab, a cotton ball, a spit, or a DNA collection kit.
[8] The method for detecting a genetic polymorphism according to any one of the [1] to [7] above, wherein the PCR primer pair is a pair of base sequences represented by the followings: SEQ ID NO: 1 and SEQ ID NO: 2, or SEQ ID NO: 3 and SEQ ID NO: 4, or SEQ ID NO: 5 and SEQ ID NO: 6, or SEQ ID NO: 7 and SEQ ID NO: 8.

| | | |
|---|---|---|
| Forward: | 5' -CAAGGAGCTGCAGGCGG-3' | (SEQ ID NO:1) |
| Reverse: | 5' - CAGCTCCTCGGTGCTCTG-3' | (SEQ ID NO:2) |
| Forward: | 5' -GGCGCAGGCCCGGCT-3' | (SEQ ID NO:3) |
| Reverse: | 5' -CGGCGCCCTCGCGG-3' | (SEQ ID NO:4) |
| Forward: | 5' -CGCAAGCTGCGTAAGCG-3' | (SEQ ID NO:5) |
| Reverse: | 5' -CGCGGATGGCGCTGAG-3' | (SEQ ID NO:6) |
| Forward: | 5' -CGTAAGCGGCTCCTCCG-3' | (SEQ ID NO:7) |
| Reverse : | 5' -CGGCGCCCTCGCGG-3' | (SEQ ID NO:8) |

[9] The method for detecting a genetic polymorphism according to any one of the [1] to [8] above, wherein, in the fluorescent labeled probe, the base sequence of the oligonucleotide that binds to the nucleic acid fragment of the apolipoprotein E gene, containing a codon encoding wild-type Cys which is the 112th amino acid residue of apolipoprotein E, and the base sequence of the oligonucleotide that binds to the nucleic acid fragment of the apolipoprotein E gene, containing a codon encoding mutant Arg which is the 112th amino acid residue of apolipoprotein E, are the base sequences represented by the following SEQ ID NO: 9 and SEQ ID NO: 10, respectively; and the base sequence of the oligonucleotide that binds to the nucleic acid fragment of the apolipoprotein E gene, containing a codon encoding wild-type Arg which is the 158th amino acid residue of apolipoprotein E, and the base sequence of the oligonucleotide that binds to the nucleic acid fragment of the apolipoprotein E gene, containing a codon encoding mutant Cys which is the 158th amino acid residue of apolipoprotein E, are the base sequences represented by the following SEQ ID NO: 11 and SEQ ID NO: 12, respectively.
5' -GGACGTGTGCGGCCG-3' · · · (SEQ ID NO:9)
5' -GGACGTGCGCGGCCG-3' · · · (SEQ ID N0:10)
5' -CTGCAGAAGCGCCTGGC-3' · · · (SEQ ID NO:11)
5' -CTGCAGAAGTGCCTGGC-3' · · · (SEQ ID N0:12)

[10] A method for determining a genetic polymorphism of apolipoprotein E present in genomic DNA collected from a subject, comprising the following steps 1 and 2:
1. a step of obtaining amplification curves based on the 112th amino acid residue and the 158th amino acid residue by the method for detecting a genetic polymorphism according to any one of the [1] to [9] above,
2.
   (a) a step of determining the genetic polymorphism to be apolipoprotein E3/E3, when a substantial increase is observed only in the fluorescence intensity derived from a fluorescent labeled probe which has an oligonucleotide that binds to a nucleic acid fragment of the apolipoprotein E gene, containing a codon encoding wild-type Cys (112th Cys probe), but not in the fluorescence intensity derived from a fluorescent labeled probe which has an oligonucleotide that binds to a nucleic acid fragment of the apolipoprotein E gene, containing a codon encoding mutant Arg (112th Arg probe), in the amplification curve based on the 112th amino acid residue obtained in the step mentioned above, and in addition,
      a substantial increase is observed only in the fluorescence intensity derived from a fluorescent labeled probe which has an oligonucleotide that binds to a nucleic acid fragment of the apolipoprotein E gene,
      containing a codon encoding wild-type Arg (158th Arg probe), but not in the fluorescence intensity derived from a fluorescent labeled probe which has an oligonucleotide that binds to a nucleic acid fragment of the apolipoprotein E gene, containing a codon encoding mutant Cys (158th Cys probe),
      in the amplification curve based on the 158th amino acid residue obtained in the step mentioned above;
   (b) a step of determining the genetic polymorphism to be apolipoprotein E2/E2, when a substantial increase is observed only in the fluorescence intensity derived from the 112th Cys probe, but not in the fluorescence intensity derived from the 112th Arg probe, in the amplification curve based on the 112th amino acid residue obtained in the step mentioned above, and in addition,
      a substantial increase is not observed in the fluorescence intensity derived from the 158th Arg probe, but observed only in the fluorescence intensity derived from the 158th Cys probe, in the amplification curve based on the 158th amino acid residue obtained in the step mentioned above;
   (c) a step of determining the genetic polymorphism to be apolipoprotein E2/E3, when a substantial increase is observed only in the fluorescence intensity derived from the 112th Cys probe, but not in the fluorescence intensity derived from the 112th Arg probe, in the amplification curve based on the 112th amino acid residue obtained in the step mentioned above, and in addition,
      a substantial increase is observed in the fluorescence intensity derived from the 158th Arg probe as well as in the fluorescence intensity derived from the 158th Cys probe, in the amplification curve based on the 158th amino acid residue obtained in the step mentioned above;
   (d) a step of determining the genetic polymorphism to be apolipoprotein E4/E4, when a substantial increase is not observed in the fluorescence intensity derived from the 112th Cys probe, but a significant increase is observed only in the fluorescence intensity derived from the 112th Arg probe, in the amplification curve based on the 112th amino acid residue obtained in the step mentioned above, and in addition,
      a substantial increase is observed only in the fluorescence intensity derived from the 158th Arg probe, but not in the fluorescence intensity derived from the 158th Cys probe, in the amplification curve based on the 158th amino acid residue obtained in the step mentioned above;
   (e) a step of determining the genetic polymorphism to be apolipoprotein E3/E4, when a substantial increase is observed both in the fluorescence intensity derived from the 112th Cys probe and in the fluorescence intensity derived from the 112th Arg probe, in the amplification curve based on the 112th amino acid residue obtained in the step mentioned above, and in addition,
      a substantial increase is observed only in the fluorescence intensity derived from the 158th Arg probe, but not in the fluorescence intensity derived from the 158th Cys probe, in the amplification curve based on the 158th amino acid residue obtained in the step mentioned above;
   (f) a step of determining the genetic polymorphism to be apolipoprotein E2/E4, when a substantial increase is observed both in the fluorescence intensity derived from the 112th Cys probe and in the fluorescence intensity derived from 112th Arg probe, in the amplification curve based on the 112th amino acid residue obtained in the step mentioned above, and in addition,
      a substantial increase is observed both in the fluorescence intensity derived from the 158th Arg probe and in the fluorescence intensity derived from the 158th Cys probe, in the amplification curve based on the 158th amino acid residue obtained in the step mentioned above.

[11] A kit for detecting or determining a genetic polymorphism of apolipoprotein E present in genomic DNA collected from a subject, comprising the following (1) to (3)
(1) a PCR enzyme,
(2) a PCR primer pair for amplifying a nucleic acid fragment of the apolipoprotein E gene, containing a codon encoding the 112th or 158th amino acid residue (Cys or Arg) of apolipoprotein E, and
(3) a set consisting of a fluorescent labeled probe which has an oligonucleotide that binds to a nucleic acid fragment of the apolipoprotein E gene, containing a codon encoding wild-type Cys which is the 112th amino acid residue of apolipoprotein E, and a fluorescent labeled probe which has an oligonucleotide that binds to a nucleic acid fragment of the apolipoprotein E gene, containing a codon encoding mutant Arg which is the 112th amino acid residue of apolipoprotein E, wherein the fluorescent dyes used for labeling are different from each other;
   or a set consisting of a fluorescent labeled probe which has an oligonucleotide that binds to a nucleic acid fragment of the apolipoprotein E gene, containing a codon encoding wild-type Arg which is the 158th amino acid residue of apolipoprotein E, and a fluorescent labeled probe which has an oligonucleotide that binds to a nucleic acid fragment of the apolipoprotein E gene, containing a codon encoding mutant Cys which is the 158th amino acid residue of apolipoprotein E, wherein the fluorescent dyes used for labeling are different from each other.

[12] The kit according to the [11] above, further comprising a surfactant and protease K.
[13] The kit according to the [12] above, wherein the surfactant is sodium dodecyl sulfate.
[14] The kit according to any one of the [11] to [13] above, further comprising Tris hydrochloric acid buffer solution containing potassium chloride, magnesium chloride, and dNTP mix.
[15] The kit according to any one of the [11] to [14] above, further comprising a substance which binds to substances that inhibit PCR, which are a biologically derived negatively charged substance that adsorbs to PCR enzymes and a biologically derived positively charged substance that adsorbs to DNA, thereby neutralizing the PCR inhibitory action of the negatively charged substance and the positively charged substance.
[16] The kit according to any one of the [11] to [14] above, further comprising a cotton swab, a cotton bud, a saliva collection tool, or a DNA collection kit.
[17] The kit according to any one of the [11] to [16] above, wherein the PCR primer pair is a pair of base sequences represented by the followings: SEQ ID NO: 1 and SEQ ID NO: 2, or SEQ ID NO: 3 and SEQ ID NO: 4.

| | | |
|---|---|---|
| Forward: | 5' -CAAGGAGCTGCAGGCGG-3' | (SEQ ID NO:1) |
| Reverse: | 5' - CAGCTCCTCGGTGCTCTG-3' | (SEQ ID NO:2) |
| Forward: | 5' -GGCGCAGGCCCGGCT-3' | (SEQ ID NO:3) |
| Reverse: | 5' -CGGCGCCCTCGCGG-3' | (SEQ ID NO:4) |
| Forward: | 5' -CGCAAGCTGCGTAAGCG-3' | (SEQ ID NO:5) |
| Reverse: | 5' -CGCGGATGGCGCTGAG-3' | (SEQ ID NO:6) |
| Forward: | 5' -CGTAAGCGGCTCCTCCG-3' | (SEQ ID NO:7) |
| Reverse: | 5' -CGGCGCCCTCGCGG-3' | (SEQ ID NO:8) |

[18] The kit according to any one of the [11] to [17] above, wherein, in the fluorescent labeled probe, the base sequence of the oligonucleotide that binds to the nucleic acid fragment of the apolipoprotein E gene, containing a codon encoding wild-type Cys which is the 112th amino acid residue of apolipoprotein E, and the base sequence of the oligonucleotide that binds to the nucleic acid fragment of the apolipoprotein E gene, containing a codon encoding mutant Arg which is the 112th amino acid residue of apolipoprotein E, are the base sequences represented by the following SEQ ID NO: 9 and SEQ ID NO: 10, respectively; and the base sequence of the oligonucleotide that binds to the nucleic acid fragment of the apolipoprotein E gene, containing a codon encoding wild-type Arg which is the 158th amino acid residue of apolipoprotein E, and the base sequence of the oligonucleotide that binds to the nucleic acid fragment of the apolipoprotein E gene, containing a codon encoding mutant Cys which is the 158th amino acid residue of apolipoprotein E, are the base sequences represented by the following SEQ ID NO: 11 and SEQ ID NO: 12, respectively.
5' -GGACGTGTGCGGCCG-3' · · · (SEQ ID NO:9)
5' -GGACGTGCGCGGCCG-3' · · · (SEQ ID N0:10)
5' -CTGCAGAAGCGCCTGGC-3' · · · (SEQ ID NO:11)
5' -CTGCAGAAGTGCCTGGC-3' · · · (SEQ ID N0:12)

[19] A dementia diagnosis system, comprising the kit according to any one of the [11] to [18] above, and MRI or CT.
[20] The dementia diagnosis system according to the [19] above, wherein the dementia is Alzheimer's disease.

### Effects of the Invention

According to the present invention, the genetic polymorphism of apolipoprotein E can be detected and determined relatively easily and quickly with little burden on the subjects.

### Brief Description of the Drawing

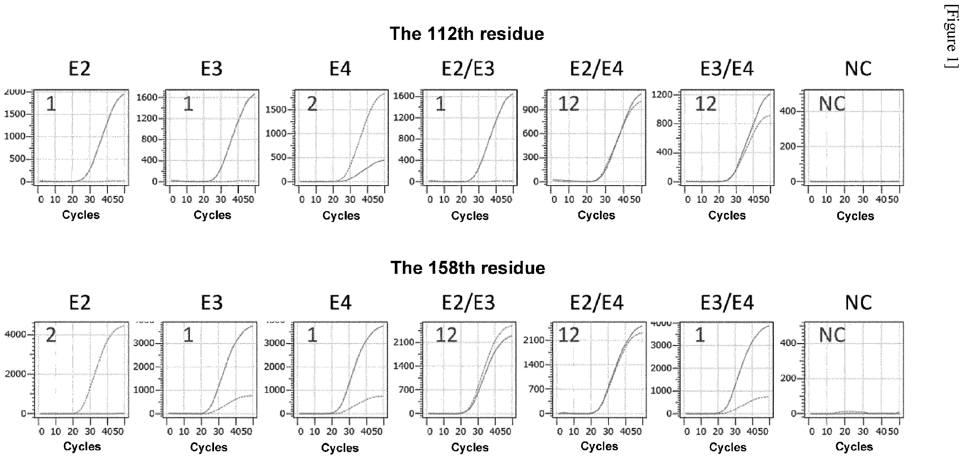

Figure 1 shows PCR amplification curves for each polymorphic genotypes of apolipoprotein E. Each upper figure represents the amplification curve corresponding to the 112th amino acid residue, and each lower figure represents the amplification curve corresponding to the 158th amino acid residue. The vertical and horizontal axes indicate the fluorescence intensity and the number of PCR cycles, respectively.

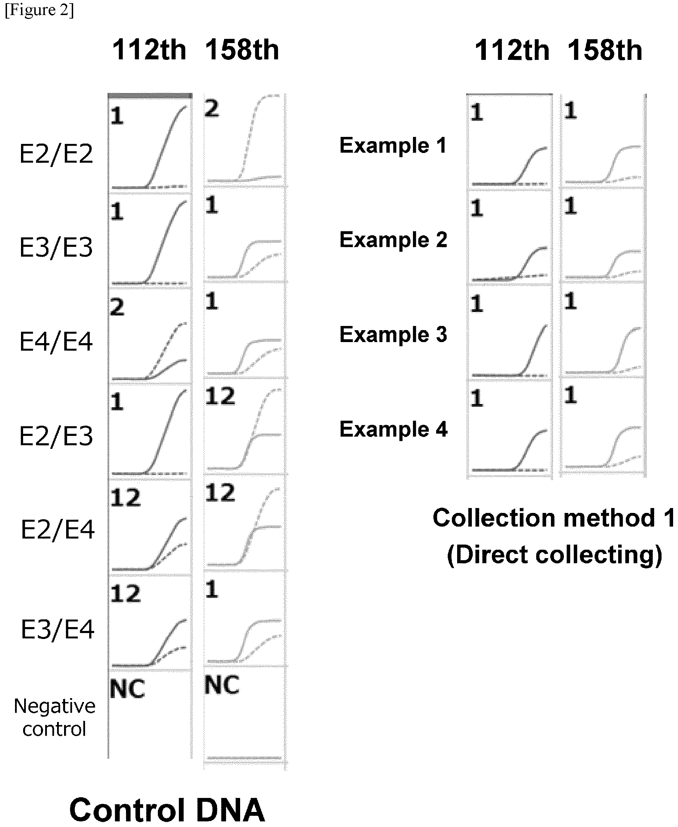

Figure 2 shows PCR amplification curves for the detection method of the present invention using direct saliva samples. Figures on the right side represent the amplification curves of Examples 1 to 4, and figures on the left side represent the amplification curves of the controls. The vertical and horizontal axes represent the fluorescence intensity and the number of PCR cycles, respectively.

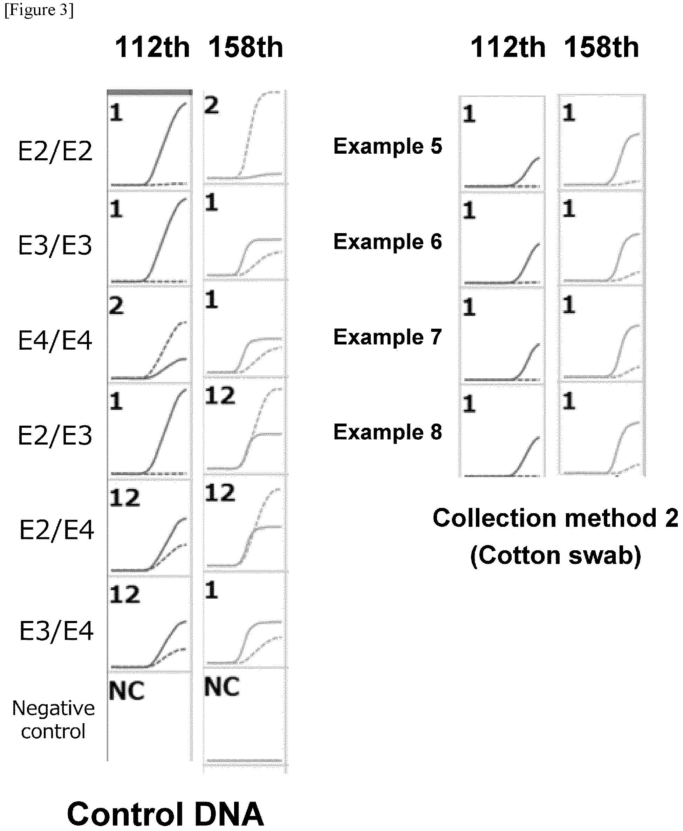

Figure 3 shows PCR amplification curves for the detection method of the present invention using saliva swab samples. Figures on the right side represent the amplification curves of Examples 1 to 4, and figures on the left side represent the amplification curves of the controls. The vertical and horizontal axes indicate the fluorescence intensity and the number of PCR cycles, respectively.

### Embodiment for Carrying out the Present Invention

The following is a detailed description of the present invention.

Apolipoprotein E, as mentioned above, has three main isoforms: ApoE2, ApoE3, and ApoE4. These differ in two amino acid residues at positions 112 and 158: ApoE2 is Cys (cysteine residue)/Cys (cysteine residue), ApoE3 is Cys (cysteine residue)/Arg (arginine residue), and ApoE4 is Arg (arginine residue)/Arg (arginine residue). Of these, ApoE3, in which the 112th amino acid residue is Cys and the 158th amino acid residue is Arg, is the wild type, and most frequent. ApoE2 and ApoE4 are mutant forms.

The codon encoding the 112th and 158th Cys of apolipoprotein E is "TGC" and the codon encoding the 112th and 158th Arg of apolipoprotein E is "CGC".

### 1 Method for detecting genetic polymorphism in accordance with the present invention

The method for detecting a genetic polymorphism in accordance with the present invention (herein referred to as "the detection method of the present invention") is a method for detecting a genetic polymorphism of apolipoprotein E present in genomic DNA collected from a subject, and is characterized by comprising the following steps 1 to 3:
1. a step of releasing a DNA from exocrine fluid or mucus containing epithelial cells collected from a subject and preparing a specimen containing the DNA;
2. a step of adding the followings to the specimen containing the DNA and then mixing:
   (1) a PCR enzyme, (2) a PCR primer pair for amplifying a nucleic acid fragment of the apolipoprotein E gene containing a codon encoding the 112th or 158th amino acid residue (Cys or Arg) of apolipoprotein E, and (3) a set consisting of a fluorescent labeled probe which has an oligonucleotide that binds to a nucleic acid fragment of the apolipoprotein E gene, containing a codon encoding wild-type Cys which is the 112th amino acid residue of apolipoprotein E, and a fluorescent labeled probe which has an oligonucleotide that binds to a nucleic acid fragment of the apolipoprotein E gene, containing a codon encoding mutant Arg which is the 112th amino acid residue of apolipoprotein E, wherein the fluorescent dyes used for labeling are different from each other; or a set consisting of a fluorescent labeled probe which has an oligonucleotide that binds to a nucleic acid fragment of the apolipoprotein E gene, containing a codon encoding wild-type Arg which is the 158th amino acid residue of apolipoprotein E, and a fluorescent labeled probe which has an oligonucleotide that binds to a nucleic acid fragment of the apolipoprotein E gene, containing a codon encoding mutant Cys which is the 158th amino acid residue of apolipoprotein E, wherein the fluorescent dyes used for labeling are different from each other; and
3. a step of performing PCR on the said mixture and measuring the fluorescence intensity from the PCR product corresponding to the 112th or 158th amino acid residue of apolipoprotein E of the said subj ect.

### 1.1 Step 1

Step 1 is a process of releasing DNA from exocrine fluid or mucus containing epithelial cells collected from a subject and preparing a specimen containing the DNA.

The exocrine fluid or mucus is not particularly restricted, but can include, for example, saliva and nasal mucus fluid. In the present invention, saliva is preferred for its simplicity. Methods of collecting saliva from a subject are not particularly restricted, but can include, for example, a method of having a subject spit out saliva directly or a method of rubbing the inside of the cheek with a cotton swab or other tool.

The amount of the exocrine fluid or mucus may be small. Specifically, for example, a range of 0.1 µL to 10 µL is appropriate, and a range of 0.5 µL to 5 µL is preferred. In case of having a subject spit out saliva as the exocrine fluid directly, saliva above the above range may be collected and the necessary amount may be used as appropriate.

The release of DNA from the exocrine fluid or mucus can be performed by a conventional method. For example, the collected exocrine fluid can be treated with a surfactant and protease K. Specifically, the collected exocrine fluid can be added to a solution containing a surfactant, sodium chloride, Tris hydrochloric acid (pH 8.0), EDTA, or a solution containing protease K in addition to them, and stirred using a vortex mixer. The temperature can be heated if necessary.

As the above surfactants, anionic, cationic, amphoteric, or nonionic surfactants can be selected as appropriate. Among these, anionic surfactants are preferred, with sodium dodecyl sulfate being more preferred. The surfactant can be used, for example, in the range of 0.1% to 0.5% (w/v).

Protease K has an action of inactivating DNA- and RNA-degrading enzymes and can be used in the range of 5 mg/mL to 20 mg/mL, for example.

### 1.2 Step 2

Step 2 is a process of preparing the so-called PCR reaction solution necessary to start the polymerase chain reaction (PCR: Polymerase Chain Reaction) by adding PCR enzymes, PCR primer pairs, fluorescent labeled probes, etc. to the specimen and mixing them.

The PCR enzymes can include, for example, DNA polymerase, which is a thermostable DNA polymerase from thermophilic bacteria, and Taq, Tth, KOD, Pfu and their mutants can be used, but DNA polymerase is not restricted to them. To avoid nonspecific amplification, hot-start DNA polymerases may be used. The hot-start DNA polymerases can include BIOTAQ (registered trademark) hot-start DNA polymerase. The hot-start DNA polymerases include DNA polymerases to which anti-DNA polymerase antibodies are attached and DNA polymerases with thermosensitive chemical modification of the enzyme active site, both of which may be used in the present invention.

The PCR primer pairs used in the detection method of the present invention can amplify a nucleic acid fragment containing a codon site encoding the 112th or 158th amino acid residue (Cys or Arg) of apolipoprotein E, the polymorphic site of the apolipoprotein E gene to be analyzed, by PCR using genomic DNA in exocrine fluid, etc. collected from a subject as the template.

As the length of the nucleic acid fragment to be amplified, a range of 20 bases to 1000 bases including the aforementioned polymorphic site of the apolipoprotein E gene is appropriate, and a range of 20 bases to 200 bases is preferred. As such PCR primer pairs, oligonucleotides (forward and reverse primers) that hybridize under stringent conditions to the region consisting of the base sequences shown in SEQ ID NO: 13 and SEQ ID NO: 14 are preferred. Stringent conditions here refer to conditions in which the binding of the primer to the template DNA is specific in the annealing in PCR, which is a step in which the primer binds to the template DNA. PCR primer pairs for the present invention are preferably 50 bases to 150 bases in length. The base sequences of the more preferred PCR primer pairs in the present invention can include, for example, the followings.

### <For genetic polymorphism corresponding to the 112th amino acid residue>

| | | |
|---|---|---|
| Forward: | 5' -CAAGGAGCTGCAGGCGG- 3' | (SEQ ID NO:1) |
| Reverse: | 5' -CAGCTCCTCGGTGCTCTG-3' | (SEQ ID NO:2) |
| Forward: | 5' -GGCGCAGGCCCGGCT-3' | (SEQ ID NO:3) |
| Reverse: | 5' -CGGCGCCCTCGCGG-3' | (SEQ ID NO:4) |

### <For genetic polymorphism corresponding to the 158th amino acid residue>

| | | |
|---|---|---|
| Forward: | 5' -CGCAAGCTGCGTAAGCG-3' | (SEQ ID NO:5) |
| Reverse: | 5' -CGCGGATGGCGCTGAG-3' | (SEQ ID NO:6) |
| Forward: | 5' -CGTAAGCGGCTCCTCCG-3' | (SEQ ID NO:7) |
| Reverse: | 5' -CGGCGCCCTCGCGG-3' | (SEQ ID NO:8) |

SEQ ID NOs: 13 and 14 below are the base sequences of the region containing the genetic polymorphism corresponding to the 112th amino acid residue and the genetic polymorphism corresponding to 158th amino acid residue, respectively, in the wild-type ApoE gene (ApoE3/E3). The parts indicated in quotation marks are, respectively, the codons corresponding to the 112th and 158th amino acid residues. An arbitrary base sequence in SEQ ID NOs: 13 and 14 can be amplified as long as it contains the polymorphic site.

5' -
GAAGGCCTACAAATCGGAACTGGAGGAACAACTGACCCCGGTG
GCGGAGGAGACGCGGGCACGGCTGTCCAAGGAGCTGCAGGCGG
CGCAGGCCCGGCTGGGCGCGGACATGGAGGACGTG"TGC"GGCC
GCCTGGTGCAGTACCGCGGCGAGGTGCAGGCCATGCTCGGCCA
GAGCACCGAGGAGCTGCGGGTGCGCCTCGCCTCCCACCTGCGC
AAGCTGCGTAAGCGGCTCCTCCGCGATGCC
-3' ・・・(SEQ ID NO:13)

5’－
GCCGCCTGGTGCAGTACCGCGGCGAGGTGCAGGCCATGCTCGG
CCAGAGCACCGAGGAGCTGCGGGTGCGCCTCGCCTCCCACCTG
CGCAAGCTGCGTAAGCGGCTCCTCCGCGATGCCGATGACCTGC
AGAAG“CGC”CTGGCAGTGTACCAGGCCGGGGCCCGCGAGGGC
GCCGAGCGCGGCCTCAGCGCCATCCGCGAGCGCCTGGG
－3’ ・・・ (SEQ ID NO:14)

The fluorescent labeled probe set used in the detection method of the present invention is a combination of a fluorescent labeled probe which has an oligonucleotide that binds to a nucleic acid fragment of the apolipoprotein E gene, containing a codon encoding wild-type Cys which is the 112th amino acid residue of apolipoprotein E (herein referred to as the " 112th Cys probe"), and a fluorescent labeled probe which has an oligonucleotide that binds to a nucleic acid fragment of the apolipoprotein E gene, containing a codon encoding mutant Arg which is the 112th amino acid residue (herein referred to as the " 112th Arg probe"); or a combination of a fluorescent labeled probe which has an oligonucleotide that binds to a nucleic acid fragment of the apolipoprotein E gene, containing a codon encoding wild-type Arg which is the 158th amino acid residue of apolipoprotein E (herein referred to as the " 158th Arg probe"), and a fluorescent labeled probe which has an oligonucleotide that binds to a nucleic acid fragment of the apolipoprotein E gene, containing a codon encoding mutant Cys which is the 158th amino acid residue (herein referred to as the "158th Cys probe").

Then, the fluorescent dyes are different from each other between the 112th Cys probe and 112th Arg probe, and between the 158th Arg probe and 158th Cys probe. This allows you to determine whether the amino acid residue at the site is Cys or Arg.

The 112th Cys and 158th Cys probes hybridize under stringent conditions to nucleic acid fragments amplified by PCR in which the 112th or 158th amino acid residue is encoded as Cys, but do not hybridize under stringent conditions to nucleic acid fragments amplified by PCR in which the 112th or 158th amino acid residue is encoded as Arg. On the other hand, the 112th Arg and 158th Arg probes hybridize under stringent conditions to nucleic acid fragments amplified by PCR in which the 112th or 158th amino acid residue is encoded as Arg, but do not hybridize under stringent conditions to nucleic acid fragments amplified by PCR in which the 112th or 158th amino acid residue is encoded as Cys.

Here, stringent conditions are defined as conditions under which specific hybrids are formed during annealing between nucleic acid fragments amplified by PCR and oligonucleotides in fluorescent labeled probes, and nonspecific hybrids are not formed.

The oligonucleotides in the fluorescent labeled probes used in the detection method of the present invention are preferably 10 to 25 bases in length, more preferably 15 to 20 bases. The most preferred base sequence of each oligonucleotide in the fluorescent labeled probe set in the detection method of the present invention is shown below. SEQ ID NO: 9 is a base sequence that binds to the nucleic acid fragment in which the 112th amino acid residue of apolipoprotein E is wild-type (Cys). Similarly, SEQ ID NOs: 10 to 12, respectively, are the base sequences that bind to the nucleic acid fragments in which the 112th amino acid residue is mutant (Arg), or the 158th amino acid residue is wild-type (Arg) or mutant (Cys).
5' -GGACGTGTGCGGCCG-3' · · · (SEQ ID NO:9)
5' -GGACGTGCGCGGCCG-3' · · · (SEQ ID N0:10)
5' -CTGCAGAAGCGCCTGGC-3' · · · (SEQ ID NO:11)
5' -CTGCAGAAGTGCCTGGC-3' · · · (SEQ ID N0:12)

The PCR reaction solution usually contains a PCR buffer. As the PCR buffer, Tris-HCl is preferred. The PCR buffer usually contains KCl, MgCh and dNTP mix (deoxyribonucleotide 5'-triphosphate; a mixture consisting of dATP, dGTP, dCTP and dTTP), but are not restricted thereto. The concentrations of dNTPs, MgClz, KCl and buffers are set appropriately by persons skilled in the art. For example, 1.5 mM for MgCl₂, 35 mM for KCl, 200 µM each for dNTPs and 10 mM for Tris-HCl.

In the detection method of the present invention, the PCR buffer can also contain a substance which binds to substances that inhibit PCR, which are a biologically derived negatively charged substance that adsorbs to a DNA polymerase (e.g., certain sugars and dyes) and a biologically derived positively charged substance that adsorbs to DNA (e.g., certain proteins), thereby neutralizing the PCR inhibitory action of the negatively charged substance and the positively charged substance.

Specifically, the PCR buffer can include, for example, the gene amplification reagent Ampdirect (registered trademark) and Ampdirect (registered trademark) Plus (both manufactured by Shimadzu Corporation).

### 1.3 Step 3

Step 3 is a process of performing PCR on the prepared sample and measuring the fluorescence intensity from the PCR product corresponding to the 112th or 158th amino acid residue of apolipoprotein E.

In the detection method of the present invention, PCR is used to detect the genetic polymorphism of apolipoprotein E. The PCR conditions (temperature, time and number of cycles) can be easily set by persons skilled in the art. In the detection method of the present invention, detection of PCR products can be performed by real-time measurement. The real-time measurement of PCR products is also called a real-time PCR.

In the detection method of the present invention, fluorescent labeled probes with oligonucleotides can be used to detect a PCR product by fluorescence. The fluorescent labeled probes can include, but are not limited to, hydrolysis probes (such as TaqMan (registered trademark) probes), Molecular Beacon and cycling probes. Among these, the hydrolysis probes are preferred from the aspects of specificity of detection and multiplex analysis. Molecular Beacon is preferred from the aspects of high sensitivity detection. From the aspects of highly accurate detection, it is preferable to use cycling probes.

The hydrolysis probes are oligonucleotides modified with a fluorescent dye at the 5' end and a quencher substance at the 3' end. The hydrolysis probe hybridizes specifically to the template DNA during PCR annealing, but because of the presence of the quencher on the probe, the generation of fluorescence is suppressed (quenched) by fluorescence resonance energy transfer (FRET) even when the probe is irradiated with excitation light. In the subsequent elongation reaction step, when the hydrolysis probe hybridized to the template DNA is degraded by the 5'→3' exonuclease activity of Taq DNA polymerase, the fluorescent dye is released from the probe and emits fluorescence through the cancellation of quenching by the quencher. By measuring the intensity of this fluorescence, the production amount of amplified product can be measured.

From the viewpoint of more accurate detection of the genetic polymorphism, the above fluorescent labeled probes may be in a form including flap endonuclease (FEN). Such fluorescent labeled probes include, for example, a molecular set including FEN, a detection probe, and a detection FRET cassette corresponding to said probe. The detection probe can be one for wild-type detection and one for mutant detection, each having a separate flap sequence at the end. The probes for wild-type detection can be structured to contain an oligonucleotide with a base sequence that hybridizes specifically to wild-type DNA, and similarly the probes for mutant detection can be structured to contain an oligonucleotide with a base sequence that hybridizes specifically to mutant DNA. The FRET cassette for detection is a molecule that recognizes each of the detection probes and can be structured to have a fluorescent dye bound to the end of the recognition site. The fluorescent dye can be quenched by the quencher in the FRET cassette.

When PCR is performed using such a fluorescent labeled probe, and the oligonucleotide and the detection probe hybridize to the template DNA in the annealing and the elongation reaction steps, a triplex structure (flap structure) composed of the oligonucleotide, the detection probe, and the template DNA can be formed. In this case, FEN cleaves the bond and releases the flap sequence at the end of the detection probe (Note that the oligonucleotide becomes the PCR amplified product through an elongation reaction step.).

The released flap sequence hybridizes to the corresponding FRET cassette, followed by the formation of a triplex (flap structure) composed of the flap sequence, the FRET cassette, and the fluorescent dye bound to it. In this case, FEN cleaves the bond and releases the fluorescent dye. The released fluorescent dye emits fluorescence through the cancellation of the quenching by FRET. By measuring the intensity of this fluorescence, the production amount of PCR amplified product can be measured.

The fluorescent dyes mentioned above include, for example, FAM (6-carboxyfluorescein), ROX (6-carboxy-X-rhodamine), Cy3 and Cy5 (Cyanine dyes), HEX (4,7,2',4',5',7'-hexachloro-6-carboxyfluorescein), and the like, but are not limited to them.

The quenchers include TAMRA (registered trademark), BHQ (Black Hole Quencher, registered trademark) 1, BHQ2, MGB-Eclipse (registered trademark), DABCYL, BHQ3, and the like, but are not limited to them.

The detection method of the present invention uses two types each of fluorescent labeling probes, namely, the 112th Cys probe and the 112th Arg probe, or the 158th Arg probe and the 158th Cys probe, and distinctively detects the two types of DNA target sequences. Accordingly, the above two types each of fluorescent labeling probes are labeled with different fluorescent dyes from each other. The combination of different fluorescent dyes is not restricted as long as they have different fluorescent properties and do not interfere with each other in the fluorescence measurement. Specifically, for example, FAM can be used for the 112th Cys probe, ROX can be used for the 112th Arg probe, FAM can be used for the 158th Arg probe, and ROX can be used for the 158th Cys probe. In other words, FAM can be used for the wild type and ROX can be used for the mutant type.

### 2 Determination of genetic polymorphism of apolipoprotein E

In the detection method of the present invention, the amplification curve of the PCR product is monitored using a fluorescent filter corresponding to the used fluorescent dye in the real-time measurement of the PCR product. If the fluorescence intensity increases substantially depending on the number of PCR cycles, the presence of the DNA to be analyzed in the sample is judged positive. On the other hand, if the fluorescence intensity does not substantially increase in PCR, a negative judgment is made.

In the present invention, the amplification curves based on the 112th amino acid residue and the 158th amino acid residue can be obtained for each polymorphic genotype of apolipoprotein E, for example, as shown in Figure 1 by the detection method of the present invention. In Figure 1, in each data based on the 112th amino acid residue, the solid line represents the fluorescence intensity derived from Cys and the dashed line represents the fluorescence intensity derived from Arg. On the other hand, in each data based on the 158th amino acid residue, the solid line represents the fluorescence intensity derived from Arg, and the dashed line represents the fluorescence intensity derived from Cys. Thus, the solid line corresponds to the amino acid residue encoded by the wild-type apolipoprotein E gene, and the dashed line corresponds to the amino acid residue encoded by the mutant apolipoprotein E gene.

Then, from the obtained amplification curve, the genetic polymorphism of the apolipoprotein E can be determined as follows.

If, in the obtained amplification curve based on the 112th amino acid residue, a substantial increase is observed only for the fluorescence intensity derived from the 112th Cys probe (the solid line in Fig. 1, the same applies hereinafter), whereas no substantial increase is observed for the fluorescence intensity derived from the 112th Arg probe (the dashed line in Fig. 1, the same applies hereinafter), and further if, in the obtained amplification curve based on the 158th amino acid residue, a substantial increase is observed only for the fluorescence intensity derived from the 158thArg probe (the solid line in Fig. 1, the same applies hereinafter), whereas no substantial increase is observed for the fluorescence intensity derived from the 158th Cys probe (the dashed line in Fig. 1, the same applies hereinafter), you can determine the genetic polymorphism of apolipoprotein E as E3/E3 (homozygous).

If, in the obtained amplification curve based on the 112th amino acid residue, a substantial increase is observed only for the fluorescence intensity derived from the 112th Cys probe, whereas no substantial increase is observed for the fluorescence intensity derived from the 112th Arg probe, and further if, in the obtained amplification curve based on the 158th amino acid residue, no substantial increase is observed for the fluorescence intensity derived from the 158th Arg probe and a substantial increase is observed only for the fluorescence intensity derived from the 158th Cys probe, you can determine the genetic polymorphism of apolipoprotein E as E2/E2 (homozygous).

If, in the obtained amplification curve based on the 112th amino acid residue, a substantial increase is observed only for the fluorescence intensity derived from the 112th Cys probe, whereas no substantial increase is observed for the fluorescence intensity derived from the 112th Arg probe, and further if, in the obtained amplification curve based on the 158th amino acid residue, a substantial increase is observed for the fluorescence intensity derived from the 158th Arg probe and a substantial increase is also observed for the fluorescence intensity the 158th Cys probe, you can determine the genetic polymorphism of apolipoprotein E as E2/E3 (heterozygous).

If, in the obtained amplification curve based on the 112th amino acid residue, no substantial increase is observed for the fluorescence intensity derived from the 112th Cys probe, whereas a significant increase is observed only for the fluorescence intensity derived from the 112th Arg probe, and further if, in the obtained amplification curve based on the 158th amino acid residue, a substantial increase is observed only for the fluorescence intensity derived from 158th Arg probe, whereas no substantial increase is observed for the fluorescence intensity derived from the 158th Cys probe, you can determine the genetic polymorphism of apolipoprotein E as E4/E4 (homozygous).

If, in the obtained amplification curve based on the 112th amino acid residue, a substantial increase is observed for both the fluorescence intensity derived from the 112th Cys probe and the fluorescence intensity derived from the 112th Arg probe, and further if, in the obtained amplification curve based on the 158th amino acid residue, a substantial increase is observed only for the fluorescence intensity derived from the 158th Arg probe, whereas no substantial increase is observed for the fluorescence intensity derived from the 158th Cys probe, you can determine the genetic polymorphism of apolipoprotein E as E3/E4 (heterozygous).

If, in the obtained amplification curve based on the 112th amino acid residue, a substantial increase is observed for both the fluorescence intensity derived from the 112th Cys probe and the fluorescence intensity derived from the 112th Arg probe, and further if, in the obtained amplification curve based on the 158th amino acid residue, a substantial increase is observed for both the fluorescence intensity derived from the 158th Arg probe and the fluorescence intensity derived from the 158th Cys probe, you can determine the genetic polymorphism of apolipoprotein E as E2/E4 (heterozygous).

Here, the term "substantial increase" in fluorescence intensity refers to an increase in fluorescence intensity that can be judged by persons skilled in the art to be derived from the probe binding to the base sequence corresponding to the amino acid residue, rather than, for example, an increase in false signal due to artifacts or other factors (e.g., a gradual increase in fluorescence due to dissociation from the quencher as the probe breaks down due to temperature).

The above determination methods can be organized as the judgment table for apolipoprotein E genetic polymorphism as shown in Table 1 below, wherein the judgment is indicated as "1" when there is a substantial increase of the fluorescence intensity only in the solid line, the judgment is indicated as "2" when there is a substantial increase only in the dashed line, and the judgment is indicated as "12" when there are substantial increases in both lines, in each of the data in Figure 1.

**[Table 1]**

| | | 112 th | | |
|---|---|---|---|---|
| | Judgment | 1 | 2 | 12 |
| 158 th | 1 | E3/E3 | E4/E4 | E3/E4 |
| | 2 | E2/E2 | | |
| | 12 | E2/E3 | | E2/E4 |

As shown in Table 1, you can determine that those wherein the judgment indications of the 112th and the 158th are 1 and 1, 1 and 2, and 2 and 1, respectively, are homozygous, and of these, the case of 1 and 1 is wild type and the rest are E2 or E4 mutant. You can determine that those wherein the judgment indications of the 112th and the 158th are 12 and 12 are heterozygous between E2 and E4 mutants, and the other combinations are E2/E3 or E3/E4 heterozygous between wild type and mutant.

### 3 Kit according to the present invention

The kit according to the present invention (hereinafter referred to as "the present invention kit") is a kit for detecting or determining a genetic polymorphism of apolipoprotein E present in genomic DNA collected from a subject, and is characterized by comprising the following (1) to (3).
(1) a PCR enzyme,
(2) a PCR primer pair for amplifying a nucleic acid fragment of the apolipoprotein E gene, containing a codon encoding the 112th or 158th amino acid residue (Cys or Arg) of apolipoprotein E, and
(3) a set consisting of the 112th Cys probe and the 112th Arg probe, wherein the fluorescent dyes used for labeling are different from each other; or a set consisting of the 158th Arg probe and the 158th Cys probe, wherein the fluorescent dyes used for labeling are different from each other.

In the present invention kit, terms such as PCR primer pairs and fluorescent labeled probe sets have the same meaning as the foregoing. The present invention kit can be used in a rapid genotype-determining system for simple and rapid genetic testing at clinical sites. The rapid genotype-determining system include, for example, the GTS-7000 manufactured by Shimadzu Corporation.

In one embodiment of the present invention, the present invention kit can comprise a PCR buffer containing a surfactant and protease K for use in specimen pretreatment to facilitate application to the rapid genotype-determining system. The surfactant is preferably an anionic surfactant, more preferably sodium dodecyl sulfate. In one embodiment, the PCR buffer may be Tris buffer containing KCl, MgCh and dNTP mix (a mixture consisting of dATP, dGTP, dCTP and dTTP). In one embodiment, the PCR buffer may contain a substance which binds to substances that inhibit PCR, which are a biologically derived negatively charged substance that adsorbs to a DNA polymerase and a biologically derived positively charged substance that adsorbs to DNA, thereby neutralizing the PCR inhibitory action of the negatively charged substance and the positively charged substance. In one embodiment, the present invention kit can comprise Ampdirect (registered trademark) or Ampdirect (registered trademark) Plus (both manufactured by Shimadzu Corporation), reagents for gene amplification.

On applying the present invention kit to the rapid genotype-determining system, incorporation of an artificially synthesized wild-type gene containing a codon encoding wild-type Cys which is the 112th amino acid residue of apolipoprotein E, and an artificially synthesized mutant gene containing a codon encoding mutant Arg which is the 112th amino acid residue thereof; and/or incorporation of an artificially synthesized wild-type gene containing a codon encoding wild-type Arg which is the 158th amino acid residue of apolipoprotein E, and an artificially synthesized mutant gene containing a codon encoding mutant Cys which is the 158th amino acid residue thereof, as a positive control, allow automated genotype determination.

The present invention can also provide a dementia diagnosis system including the present invention kit and MRI and/or CT. Combining the present invention kit or the detection method of the present invention using the kit with MRI and/or CT increases the accuracy of dementia diagnosis. The dementia can include, for example, Alzheimer's disease.

### Example

Hereinafter, the present invention will be described showing Examples, but the present invention is not limited in any way to the invention shown below.

### [Measurement of ApoE genetic polymorphism using saliva samples]

Using saliva samples from humans (4 subjects), the accuracy of the ApoE genetic polymorphism determination was confirmed based on the present invention kit and the detection method of the present invention.

The reagents used were as follows.
- Cell lysate: prepared by mixing Tris hydrochloric acid (pH 8.0, 20 mM), EDTA (5 mM), NaCl (400 mM), SDS (0.3%), and distilled water (each concentration indicates the final concentration of each component).
- PCR buffer: Ampdirect (registered trademark) Plus reagent (manufactured by Shimadzu Corporation)
- Enzyme: BIOTAQ (registered trademark) DNA polymerase (manufactured by Bioline)
- 112-PT: Primer (SEQ ID Nos: 1 and 2) / Fluorescent labeled probe (containing oligonucleotides of SEQ ID Nos: 9 and 10), mixed solution
- 158-PT: Primer (SEQ ID Nos: 5 and 6) / Fluorescent labeled probe (containing oligonucleotides of SEQ ID Nos: 11 and 12), mixed solution
- E2/E2: ApoE2/ApoE2 control DNA
- E3/E3: ApoE3/ApoE3 control DNA
- E4/E4: ApoE4/ApoE4 control DNA
- E2/E3: ApoE2/ApoE3 control DNA
- E2/E4: ApoE2/ApoE4 control DNA
- E3/E4: ApoE3/ApoE4 control DNA

Note that a hydrolysis probe (TaqMan (registered trademark) probe) was used for each of the above fluorescent labeled probes. The fluorescent dye was FAM for the probes with an oligonucleotide of SEQ ID No: 9 or 11, and was ROX for the probes with an oligonucleotide of SEQ ID No: 10 or 12.

### [Examples 1 to 4] Measurement using directly collected samples (collection method 1)

The samples were 2 µL each of human (4 subjects) saliva spit directly into microtubes (1.5 mL).

The PCR reaction solution for the detection of the polymorphism corresponding to the 112th amino acid residue of the ApoE-encoded protein was prepared by mixing PCR buffer, Enzyme, 112-PT, and purified water. An aliquot of 24 µL of the PCR reaction solution was dispensed into a PCR reaction tube, and 1 µL of each of the above saliva samples was added to the tube and mixed. The PCR reaction solution for detection of the polymorphism corresponding to the 158th amino acid residue was also prepared by mixing PCR buffer, Enzyme, 158-PT, and purified water. The reaction solution was also dispensed in 24 µL aliquots in the same manner, and 1 µL of each of the above saliva samples was added and mixed.

PCR reactions were immediately measured for each mixture obtained using a real-time PCR system (GTS-7000, Shimadzu Corporation) (Examples 1 to 4). In the measurement, the enzyme was first activated at 95°C/10 min, followed by 50 cycles of PCR at 95°C/10 sec to 60°C/30 sec.

Note that as positive and negative controls, mixtures respectively added with each of the above E2/E2 to E3/E4 DNA or distilled water, instead of saliva samples, were prepared, and PCR reactions were measured in the same manner.

The measurement results are shown in Figure 2. In any of Example 1 to 4, a substantial amplification was observed only for the fluorescence intensity derived from the 112th Cys probe (solid line), and no amplification was observed for the fluorescence intensity derived from the 112th Arg probe (dashed line). Also, for the 158th, a substantial amplification was observed only for the fluorescence intensity derived from the 158th Arg probe (solid line), and no substantial amplification was observed for the fluorescence intensity derived from the 158th Cys probe (dashed line). From these results, each of the Examples could be determined to be E3/E3 (homozygous).

### [Examples 5 to 8] Measurement using swab samples (collection method 2)

The samples used were saliva and oral epithelial cells from humans (the same four subjects as in Examples 1 to 4) collected by cotton swab. Specifically, a cotton swab was rubbed on the inside of each subject's cheek, immersed in 100 µL of a cell lysing solution in a microtube (1.5 mL), and mixed for 20 seconds. Two µL of the lysate after mixing was used as the sample.

The mixture was prepared in the same manner as in Examples 1 to 4 above, and PCR reactions were measured under the same conditions. Positive and negative controls are prepared in the same way as above.

The measurement results are shown in Figure 3. In any of Examples 5 to 8, a substantial amplification was observed only for the fluorescence intensity derived from the 112th Cys probe (solid line), and no amplification was observed for the fluorescence intensity derived from the 112th Arg probe (dashed line). Also, for the 158th, a substantial amplification was observed only for the fluorescence intensity derived from the 158th Arg probe (solid line), and no substantial amplification was observed for the fluorescence intensity derived from the 158th Cys probe (dashed line). From these results, each of the Examples could be determined to be E3/E3 (homozygous).

From the above, it was shown that the ApoE polymorphism can be accurately identified by using the present invention kit and the detection method of present invention. In each of the above Examples, the amplification curve began to intensify at about 30 minutes after the start of the reaction, and the rise in fluorescence intensity was earlier than when measuring ordinary blood samples. It can be said that the detection method of the present invention can determine the genetic polymorphism more quickly than the conventional blood-based detection method.

### Free text for Sequence listing

SEQ ID No 1: PCR forward primer for amplifying a nucleic acid fragment of the apolipoprotein E gene, containing a codon encoding the 112th amino acid residue of apolipoprotein E.
SEQ ID No 2: PCR reverse primer for amplifying a nucleic acid fragment of the apolipoprotein E gene, containing a codon encoding the 112th amino acid residue of apolipoprotein E.
SEQ ID No 3: PCR forward primer for amplifying a nucleic acid fragment of the apolipoprotein E gene, containing a codon encoding the 112th amino acid residue of apolipoprotein E.
SEQ ID No 4: PCR reverse primer for amplifying a nucleic acid fragment of the apolipoprotein E gene, containing a codon encoding the 112th amino acid residue of apolipoprotein E.
SEQ ID No 5: PCR forward primer for amplifying a nucleic acid fragment of the apolipoprotein E gene, containing a codon encoding the 158th amino acid residue of apolipoprotein E.
SEQ ID No 6: PCR reverse primer for amplifying a nucleic acid fragment of the apolipoprotein E gene, containing a codon encoding the 158th amino acid residue of apolipoprotein E.
SEQ ID No 7: PCR forward primer for amplifying a nucleic acid fragment of the apolipoprotein E gene, containing a codon encoding the 158th amino acid residue of apolipoprotein E.
SEQ ID No 8: PCR reverse primer for amplifying a nucleic acid fragment of the apolipoprotein E gene, containing a codon encoding the 158th amino acid residue of apolipoprotein E.
SEQ ID No 9: A probe for detecting an amplified nucleic acid fragment of the apolipoprotein E gene, containing a codon for the wild-type 112th amino acid residue of apolipoprotein E.
SEQ ID No 10: A probe for detecting an amplified nucleic acid fragment of the apolipoprotein E gene, containing a codon for the mutant 112th amino acid residue of apolipoprotein E.
SEQ ID No 11: A probe for detecting an amplified nucleic acid fragment of the apolipoprotein E gene, containing a codon for the wild-type 158th amino acid residue of apolipoprotein E.
SEQ ID No 12: A probe for detecting an amplified nucleic acid fragment of the apolipoprotein E gene, containing a codon for the mutant 158th amino acid residue of apolipoprotein E.
SEQ ID No 13: The base sequence of the region containing the genetic polymorphism corresponding to the 112th amino acid residue in the ApoE gene.
SEQ ID No 13: The part from the 122nd base to the 124th base in the sequence is the wild-type codon corresponding to the 112th amino acid residue of apolipoprotein E.
SEQ ID No 14: The base sequence of the region containing the genetic polymorphism corresponding to the 158th amino acid residue in the ApoE gene.
SEQ ID No 14: The part from the 135th base to the 137th base in the sequence is the wild-type codon corresponding to the 158th amino acid residue of apolipoprotein E.

## Claims

1. A method for detecting a genetic polymorphism of apolipoprotein E present in genomic DNA collected from a subject, comprising the following steps 1 to 3:
1. a step of releasing a DNA from exocrine fluid or mucus containing epithelial cells collected from a subject and preparing a specimen containing the DNA;
2. a step of adding the followings to the specimen containing the DNA and then mixing:
(1) a PCR enzyme,
(2) a PCR primer pair for amplifying a nucleic acid fragment of the apolipoprotein E gene containing a codon encoding the 112th or 158th amino acid residue (Cys or Arg) of apolipoprotein E, and
(3) a set consisting of a fluorescent labeled probe which has an oligonucleotide that binds to a nucleic acid fragment of the apolipoprotein E gene, containing a codon encoding wild-type Cys which is the 112th amino acid residue of apolipoprotein E, and a fluorescent labeled probe which has an oligonucleotide that binds to a nucleic acid fragment of the apolipoprotein E gene, containing a codon encoding mutant Arg which is the 112th amino acid residue of apolipoprotein E, wherein the fluorescent dyes used for labeling are different from each other,
or a set consisting of a fluorescent labeled probe which has an oligonucleotide that binds to a nucleic acid fragment of the apolipoprotein E gene, containing a codon encoding wild-type Arg which is the 158th amino acid residue of apolipoprotein E, and a fluorescent labeled probe which has an oligonucleotide that binds to a nucleic acid fragment of the apolipoprotein E gene, containing a codon encoding mutant Cys which is the 158th amino acid residue of apolipoprotein E, wherein the fluorescent dyes used for labeling are different from each other; and
3. a step of performing PCR on the said mixture and measuring the fluorescence intensity from the PCR product corresponding to the 112th or 158th amino acid residue of apolipoprotein E of the said subject.

2. The method for detecting a genetic polymorphism according to Claim 1, wherein DNA is released by using a surfactant and protease K in the step 1.

3. The method for detecting a genetic polymorphism according to Claim 2, wherein the surfactant is sodium dodecyl sulfate.

4. The method for detecting a genetic polymorphism according to any one of Claims 1 to 3, wherein Tris hydrochloric acid buffer solution containing potassium chloride, magnesium chloride, and dNTP mix is further added and mixed with the above specimen.

5. The method for detecting a genetic polymorphism according to any one of Claims 1 to 4, further comprising a step of adding and mixing with the above specimen a substance which binds to substances that inhibit PCR, which are a biologically derived negatively charged substance that adsorbs to PCR enzymes and a biologically derived positively charged substance that adsorbs to DNA, thereby neutralizing the PCR inhibitory action of the negatively charged substance and the positively charged substance.

6. The method for detecting a genetic polymorphism according to any one of Claims 1 to 5, wherein the exocrine fluid or mucus is saliva.

7. The method for detecting a genetic polymorphism according to Claim 6, wherein the saliva is collected by a cotton swab, a cotton ball, a spit, or a DNA collection kit.

8. The method for detecting a genetic polymorphism according to any one of Claims 1 to 7, wherein the PCR primer pair is a pair of base sequences represented by the followings: SEQ ID NO: 1 and SEQ ID NO: 2, or SEQ ID NO: 3 and SEQ ID NO: 4, or SEQ ID NO: 5 and SEQ ID NO: 6, or SEQ ID NO: 7 and SEQ ID NO: 8.
| | | |
|---|---|---|
| Forward: | 5' -CAAGGAGCTGCAGGCGG-3' | (SEQ ID NO:1) |
| Reverse: | 5' - CAGCTCCTCGGTGCTCTG-3' | (SEQ ID NO:2) |
| Forward: | 5' -GGCGCAGGCCCGGCT-3' | (SEQ ID NO:3) |
| Reverse: | 5' -CGGCGCCCTCGCGG-3' | (SEQ ID NO:4) |
| Forward: | 5' -CGCAAGCTGCGTAAGCG-3' | (SEQ ID NO:5) |
| Reverse: | 5' -CGCGGATGGCGCTGAG-3' | (SEQ ID NO:6) |
| Forward: | 5' -CGTAAGCGGCTCCTCCG-3' | (SEQ ID NO:7) |
| Reverse: | 5' -CGGCGCCCTCGCGG-3' | (SEQ ID NO: 8) |

9. The method for detecting a genetic polymorphism according to any one of Claims 1 to 8, wherein, in the fluorescent labeled probe, the base sequence of the oligonucleotide that binds to the nucleic acid fragment of the apolipoprotein E gene, containing a codon encoding wild-type Cys which is the 112th amino acid residue of apolipoprotein E, and the base sequence of the oligonucleotide that binds to the nucleic acid fragment of the apolipoprotein E gene, containing a codon encoding mutant Arg which is the 112th amino acid residue of apolipoprotein E, are the base sequences represented by the following SEQ ID NO: 9 and SEQ ID NO: 10, respectively; and the base sequence of the oligonucleotide that binds to the nucleic acid fragment of the apolipoprotein E gene, containing a codon encoding wild-type Arg which is the 158th amino acid residue of apolipoprotein E, and the base sequence of the oligonucleotide that binds to the nucleic acid fragment of the apolipoprotein E gene, containing a codon encoding mutant Cys which is the 158th amino acid residue of apolipoprotein E, are the base sequences represented by the following SEQ ID NO: 11 and SEQ ID NO: 12, respectively.
5' -GGACGTGTGCGGCCG-3' · · · (SEQ ID NO:9)
5' -GGACGTGCGCGGCCG-3' · · · (SEQ ID NO:10)
5' -CTGCAGAAGCGCCTGGC-3' · · · (SEQ ID NO:11)
5' -CTGCAGAAGTGCCTGGC-3' · · · (SEQ ID N0:12)

10. A method for determining a genetic polymorphism of apolipoprotein E present in genomic DNA collected from a subject, comprising the following steps 1 and 2:
1. a step of obtaining amplification curves based on the 112th amino acid residue and the 158th amino acid residue by the method for detecting a genetic polymorphism according to any one of Claims 1 to 9,
2.
(a) a step of determining the genetic polymorphism to be apolipoprotein E3/E3, when a substantial increase is observed only in the fluorescence intensity derived from a fluorescent labeled probe which has an oligonucleotide that binds to a nucleic acid fragment of the apolipoprotein E gene, containing a codon encoding wild-type Cys (112th Cys probe), but not in the fluorescence intensity derived from a fluorescent labeled probe which has an oligonucleotide that binds to a nucleic acid fragment of the apolipoprotein E gene, containing a codon encoding mutant Arg (112th Arg probe), in the amplification curve based on the 112th amino acid residue obtained in the step 1, and in addition,
a substantial increase is observed only in the fluorescence intensity derived from a fluorescent labeled probe which has an oligonucleotide that binds to a nucleic acid fragment of the apolipoprotein E gene, containing a codon encoding wild-type Arg (158th Arg probe), but not in the fluorescence intensity derived from a fluorescent labeled probe which has an oligonucleotide that binds to a nucleic acid fragment of the apolipoprotein E gene, containing a codon encoding mutant Cys (158th Cys probe), in the amplification curve based on the 158th amino acid residue obtained in the step 1;
(b) a step of determining the genetic polymorphism to be apolipoprotein E2/E2, when a substantial increase is observed only in the fluorescence intensity derived from the 112th Cys probe, but not in the fluorescence intensity derived from the 112th Arg probe, in the amplification curve based on the 112th amino acid residue obtained in the step 1, and in addition,
a substantial increase is not observed in the fluorescence intensity derived from the 158th Arg probe, but observed only in the fluorescence intensity derived from the 158th Cys probe, in the amplification curve based on the 158th amino acid residue obtained in the step 1;
(c) a step of determining the genetic polymorphism to be apolipoprotein E2/E3, when a substantial increase is observed only in the fluorescence intensity derived from the 112th Cys probe, but not in the fluorescence intensity derived from the 112th Arg probe, in the amplification curve based on the 112th amino acid residue obtained in the step mentioned 1, and in addition,
a substantial increase is observed in the fluorescence intensity derived from the 158th Arg probe as well as in the fluorescence intensity derived from the 158th Cys probe, in the amplification curve based on the 158th amino acid residue obtained in the step 1;
(d) a step of determining the genetic polymorphism to be apolipoprotein E4/E4, when a substantial increase is not observed in the fluorescence intensity derived from the 112th Cys probe, but a significant increase is observed only in the fluorescence intensity derived from the 112th Arg probe, in the amplification curve based on the 112th amino acid residue obtained in the step 1, and in addition, a substantial increase is observed only in the fluorescence intensity derived from the 158th Arg probe, but not in the fluorescence intensity derived from the 158th Cys probe, in the amplification curve based on the 158th amino acid residue obtained in the step 1;
(e) a step of determining the genetic polymorphism to be apolipoprotein E3/E4, when a substantial increase is observed both in the fluorescence intensity derived from the 112th Cys probe and in the fluorescence intensity derived from the 112th Arg probe, in the amplification curve based on the 112th amino acid residue obtained in the step 1, and in addition,
a substantial increase is observed only in the fluorescence intensity derived from the 158th Arg probe, but not in the fluorescence intensity derived from the 158th Cys probe, in the amplification curve based on the 158th amino acid residue obtained in the step 1;
(f) a step of determining the genetic polymorphism to be apolipoprotein E2/E4, when a substantial increase is observed both in the fluorescence intensity derived from the 112th Cys probe and in the fluorescence intensity derived from 112th Arg probe, in the amplification curve based on the 112th amino acid residue obtained in the step 1, and in addition,
a substantial increase is observed both in the fluorescence intensity derived from the 158th Arg probe and in the fluorescence intensity derived from the 158th Cys probe, in the amplification curve based on the 158th amino acid residue obtained in the step 1.

11. A kit for detecting or determining a genetic polymorphism of apolipoprotein E present in genomic DNA collected from a subject, comprising the following (1) to (3)
(1) a PCR enzyme,
(2) a PCR primer pair for amplifying a nucleic acid fragment of the apolipoprotein E gene, containing a codon encoding the 112th or 158th amino acid residue (Cys or Arg) of apolipoprotein E, and
(3) a set consisting of a fluorescent labeled probe which has an oligonucleotide that binds to a nucleic acid fragment of the apolipoprotein E gene, containing a codon encoding wild-type Cys which is the 112th amino acid residue of apolipoprotein E, and a fluorescent labeled probe which has an oligonucleotide that binds to a nucleic acid fragment of the apolipoprotein E gene, containing a codon encoding mutant Arg which is the 112th amino acid residue of apolipoprotein E, wherein the fluorescent dyes used for labeling are different from each other;
or a set consisting of a fluorescent labeled probe which has an oligonucleotide that binds to a nucleic acid fragment of the apolipoprotein E gene, containing a codon encoding wild-type Arg which is the 158th amino acid residue of apolipoprotein E, and a fluorescent labeled probe which has an oligonucleotide that binds to a nucleic acid fragment of the apolipoprotein E gene, containing a codon encoding mutant Cys which is the 158th amino acid residue of apolipoprotein E, wherein the fluorescent dyes used for labeling are different from each other.

12. The kit according to Claim 11, further comprising a surfactant and protease K.

13. The kit according to Claim 12, wherein the surfactant is sodium dodecyl sulfate.

14. The kit according to any one of Claims 11 to 13, further comprising Tris hydrochloric acid buffer solution containing potassium chloride, magnesium chloride, and dNTP mix.

15. The kit according to any one of Claims 11 to 14, further comprising a substance which binds to substances that inhibit PCR, which are a biologically derived negatively charged substance that adsorbs to PCR enzymes and a biologically derived positively charged substance that adsorbs to DNA, thereby neutralizing the PCR inhibitory action of the negatively charged substance and the positively charged substance.

16. The kit according to any one of Claims 11 to 14, further comprising a cotton swab, a cotton bud, a saliva collection tool, or a DNA collection kit.

17. The kit according to any one of Claims 11 to 16, wherein the PCR primer pair is a pair of base sequences represented by the followings: SEQ ID NO: 1 and SEQ ID NO: 2, or SEQ ID NO: 3 and SEQ ID NO: 4.
| | | |
|---|---|---|
| Forward: | 5' -CAAGGAGCTGCAGGCGG-3' | (SEQ ID NO:1) |
| Reverse: | 5' -CAGCTCCTCGGTGCTCTG-3' | (SEQ ID NO:2) |
| Forward: | 5' -GGCGCAGGCCCGGCT-3' | (SEQ ID NO:3) |
| Reverse: | 5' -CGGCGCCCTCGCGG-3' | (SEQ ID NO:4) |
| Forward: | 5' -CGCAAGCTGCGTAAGCG-3' | (SEQ ID NO:5) |
| Reverse: | 5' -CGCGGATGGCGCTGAG-3' | (SEQ ID NO:6) |
| Forward: | 5' -CGTAAGCGGCTCCTCCG-3' | (SEQ ID NO:7) |
| Reverse: | 5' -CGGCGCCCTCGCGG-3' | (SEQ ID NO:8) |

18. The kit according to any one of Claims 11 to 17, wherein, in the fluorescent labeled probe, the base sequence of the oligonucleotide that binds to the nucleic acid fragment of the apolipoprotein E gene, containing a codon encoding wild-type Cys which is the 112th amino acid residue of apolipoprotein E, and the base sequence of the oligonucleotide that binds to the nucleic acid fragment of the apolipoprotein E gene, containing a codon encoding mutant Arg which is the 112th amino acid residue of apolipoprotein E, are the base sequences represented by the following SEQ ID NO: 9 and SEQ ID NO: 10, respectively; and the base sequence of the oligonucleotide that binds to the nucleic acid fragment of the apolipoprotein E gene, containing a codon encoding wild-type Arg which is the 158th amino acid residue of apolipoprotein E, and the base sequence of the oligonucleotide that binds to the nucleic acid fragment of the apolipoprotein E gene, containing a codon encoding mutant Cys which is the 158th amino acid residue of apolipoprotein E, are the base sequences represented by the following SEQ ID NO: 11 and SEQ ID NO: 12, respectively.
5' -GGACGTGTGCGGCCG-3' · · · (SEQ ID NO:9)
5' -GGACGTGCGCGGCCG-3' · · · (SEQ ID NO:10)
5' -CTGCAGAAGCGCCTGGC-3' · · · (SEQ ID NO:11)
5' -CTGCAGAAGTGCCTGGC-3' · · · (SEQ ID N0:12)

19. A dementia diagnosis system, comprising the kit according to any one of Claims 11 to 18, and MRI or CT.

20. The dementia diagnosis system according to Claim 19, wherein the dementia is Alzheimer's disease.
